# EUROPEAN PATENT APPLICATION

(11) **EP 4 781 906 A1**
(43) Date of publication of application: **29.07.2026**
(21) Application number: 26154816.8
(22) Date of filing: 28.01.2026
(51) Int. Cl.: A61B 5/0538, A61B 5/08, A61B 5/00, A61B 5/11, A61B 5/318

(54) **SYSTEM INCLUDING IMPLANTABLE MEDICAL DEVICE CONFIGURED TO DETECT AND IDENTIFY TYPES OF COUGH**

(30) Priority: 28.01.2025 US 202563750541 P; 21.01.2026 US 202619454781
(71) Applicant: Medtronic, Inc., Minneapolis, MN 55432 (US)
(72) Inventor: Malin, Anna J., Minneapolis, 55432 (US); Zielinski, Todd M., Minneapoplis, 55432 (US); Gautham, Rajagopal, Minneapolis, 55432 (US); Pronovici, Juliana E., Minneapolis, 55432 (US); Condie, Catherine R., Minneapolis, 55432 (US); Krause, Paul G., Minneapolis, 55432 (US); Steckler, Marc C., Minneapolis, 55432 (US); Arnebeck, Kate, Minneapolis, 55432 (US); Fischer, Trent M., Minneapolis, 55432 (US); Galarneau-Becker, Ashley, Minneaoplis, 55432 (US); Cho, Yong K., Minneapolis, 55432 (US); Sarkar, Shantanu, Minneapolis, 55432 (US)
(74) Representative: Zimmermann & Partner Patentanwälte mbB

(57) **Abstract**

An example system includes at least one implantable medical device configured to measure impedance and sense at least one or more physiological signals of a patient; and processing circuitry configured to: determine an occurrence of a cough of the patient based on at least one of the physiological signals; determine a classification of the cough based on the measured impedance; and output an indication of the classification of the cough.

## Description

This application claims the benefit of U.S. Provisional Patent Application Serial No. 63/750,541, filed January 28, 2025, the entire content of which is incorporated herein by reference.

### FIELD

This disclosure generally relates to medical device systems and, more particularly, medical device systems configured to monitor patient parameters.

### BACKGROUND

Some types of medical devices may be used to monitor one or more physiological parameters of a patient. Such medical devices may include, or may be part of a system that includes, sensors that detect signals associated with such physiological parameters. Values determined based on such signals may be used to assist in detecting changes in patient conditions, in evaluating the efficacy of a therapy, or in generally evaluating patient health.

### SUMMARY

Breathing health events or conditions, such as Chronic Obstructive Pulmonary Disease (COPD), may have occurrences or exacerbations that may be difficult to predict and/or detect, and a delay in addressing or treating such breathing health events may lead to a patient seeking emergency care. A patient having a wet cough may indicate a patient is coughing due to breathing condition such as COPD while a patient having a dry cough may indicate is coughing due to other irritations, such as due to a common cold, smoking, etc.

In general, this disclosure describes a system comprising processing circuitry and one or more implantable medical devices (IMDs) configured to sense one or more physiological signals of a patient, e.g., to measure impedance. In general, this disclosure further describes techniques for processing circuitry of the system to determine an occurrence of a cough and determine a classification of the cough based on impedance, and in some cases other physiological parameters, measured by an IMD.

For example, processing circuitry of the system may determine whether a cough is a wet cough or a dry cough based on the measured impedance. In some examples, the improved ability of the system to remotely and timely determine whether cough(s) are a wet cough or dry cough and output an indication of the classification of the cough(s) may lead to improved timely care for a patient suffering from breathing health events, such as enabling timely care to reduce the exacerbation of COPD. In some examples, processing circuitry may provide the classification of detected coughs as input to determine a health event risk score, such as a COPD risk score that indicates a determine or predict an occurrence of a health event such as COPD, which may lead to improved more timely care for the patient suffering from or predicted to suffer from a breathing health event or condition, such as enabling more timely care to reduce an exacerbation of COPD.

In one example, this disclosure describes a system comprising: at least one implantable medical device configured to measure impedance and sense at least one or more physiological signals of a patient; and processing circuitry configured to: determine an occurrence of a cough of the patient based on at least one of the physiological signals; determine a classification of the cough based on the measured impedance; and output an indication of the classification of the cough.

In another example, this disclosure describes a method comprising: measuring, by an implantable medical device (IMD), impedance of a patient; sensing, by the IMD, at least one or more physiological signals of the patient; determining, by processing circuitry, an occurrence of a cough of the patient based on at least one of the physiological signals; determining, by the processing circuitry, a classification of the cough based on the measured impedance; and outputting, by the processing circuitry, an indication of the classification of the cough.

This summary is intended to provide an overview of the subject matter described in this disclosure. It is not intended to provide an exclusive or exhaustive explanation of the apparatus and methods described in detail within the accompanying drawings and description below. Further details of one or more examples are set forth in the accompanying drawings and the description below.

### BRIEF DESCRIPTION OF DRAWINGS

FIGS. 1A-1B illustrate the environment of example medical device systems in conjunction with a patient, in accordance with one or more techniques of this disclosure.
FIG. 2 is a block diagram illustrating an example system configured to determine a classification of a cough of a patient in accordance with one or more techniques of this disclosure.
FIGS. 3A and 3B are conceptual diagrams illustrating example implantable medical devices that operate in accordance with one or more techniques of this disclosure.
FIG. 4 is a block diagram illustrating an example configuration of an implantable medical device that operates in accordance with one or more techniques of this disclosure.
FIG. 5 is a block diagram illustrating an example configuration of an external device that operates in accordance with one or more techniques of this disclosure.
FIGS. 6A-6C are flow diagrams illustrating example operations for determining an occurrence of a cough and determining a classification of the cough.

Like reference characters refer to like elements throughout the figures and description.

### DETAILED DESCRIPTION

Implantable medical devices (IMDs) are configured to monitor health based on measured impedance and at least one or more other physiological signals, such as sensed electrocardiograms (ECGs), an accelerometer signal, and/or, in some cases, other physiological signals, and detect health events or health event risk scores, such as indications of Chronic Obstructive Pulmonary Disease (COPD), a COPD risk score, episodes of arrhythmia, cardiac arrest, myocardial infarction, stroke, and seizure. Example IMDs include pacemakers and implantable cardioverter-defibrillators, which may be coupled to intravascular or extravascular leads, as well as pacemakers with housings configured for implantation within the heart, which may be leadless, such as the Micra^{™} leadless pacemaker, available from Medtronic, Inc. Some IMDs do not provide therapy, such as implantable patient monitors. One example of such an IMD is the Reveal LINQ^{™} or LINQ II^{™} insertable cardiac monitors (ICMs), available from Medtronic, Inc., which may be inserted subcutaneously. Such IMDs may facilitate relatively longer-term continuous monitoring of patients during normal daily activities, and may periodically or on demand transmit collected data, e.g., episode data for detected wet cough(s) or detected COPD, to a remote patient monitoring system, such as the Medtronic CareLink^{™} Network via a home monitoring system or a smart phone application.

Different types of coughs may be a sign of a particular patient condition and/or a change in a patient condition. An increased coughing frequency detected in a patient may indicate an exacerbation in need of one or more medical treatments. For example, an indication of wet coughs, such as chronic wet coughing, may provide an important metric for monitoring one or more patient conditions such as COPD. Data collected by an IMD, or one or more implantable or external devices, may be used detect coughs and determine a classification of the coughs, such as whether the detected coughs are wet coughs or dry coughs. For example, an IMD, or one or more other devices, may be configured to record an electrogram (EGM) signal and an accelerometer signal, both of which may include information that may be analyzed to detect coughs, and be configured to measure impedance to determine a classification of the cough based on the measured impedance.

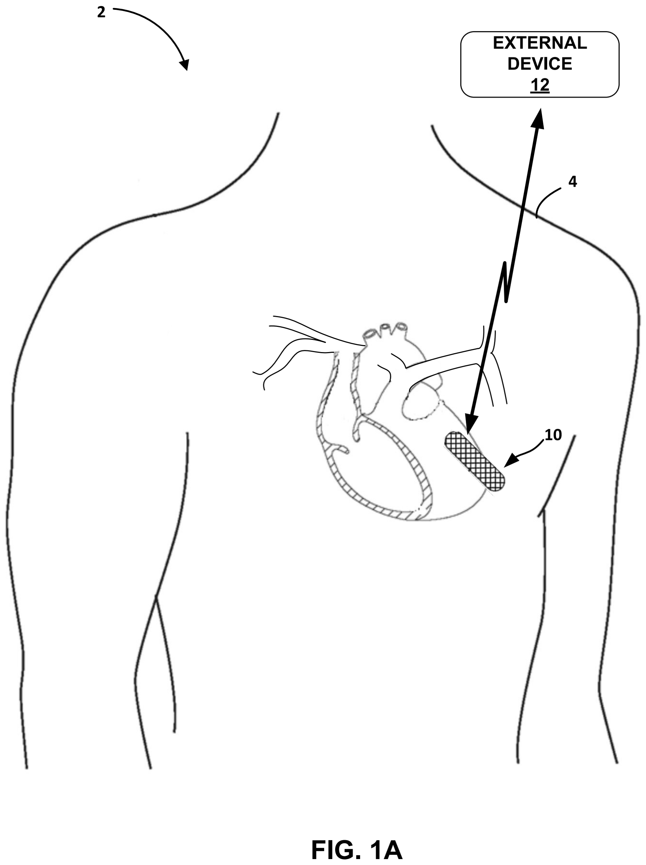

FIG. 1A illustrates the environment of an example medical device system 2 in conjunction with a patient 4, in accordance with one or more techniques of this disclosure. The example techniques may be used with one or more patient sensing devices, e.g., including an IMD 10, which may be in wireless communication with one or more computing devices, e.g., external device 12. Although not illustrated in FIG. 1A, IMD 10 includes electrodes and/or other sensors to sense physiological signals of patient 4 and may collect and store metric values based on the sensed signals. In some examples, IMD 10 may be configured to measure impedance and sense one or more physiological signals of patient. 4, such as an ECG signal and/or an accelerometer signal. One or more elements of system 2 may determine an occurrence of a cough of patient 4 based on the one or more physiological signals and may determine a classification of the cough, such as the cough being a wet cough or dry cough, based on the impedance measured by IMD 10.

IMD 10 may be implanted outside of a thoracic cavity of patient 4 (e.g., subcutaneously in the pectoral location illustrated in FIG. 1A). IMD 10 may be positioned near the sternum near or just below the level of the heart of patient 4, e.g., at least partially within the cardiac silhouette, and be configured to sense an ECG and/or other physiological signals from that position. In some examples, IMD 10 takes the form of the Reveal LINQ^{™} or LINQ II^{™} ICM. In some examples, IMD 10 includes additional sensors, such as one or more sensors configured to sense patient activity and/or posture, e.g., one or more accelerometers, heart sounds, respiration, blood pressure (BP), or oxygen saturation.

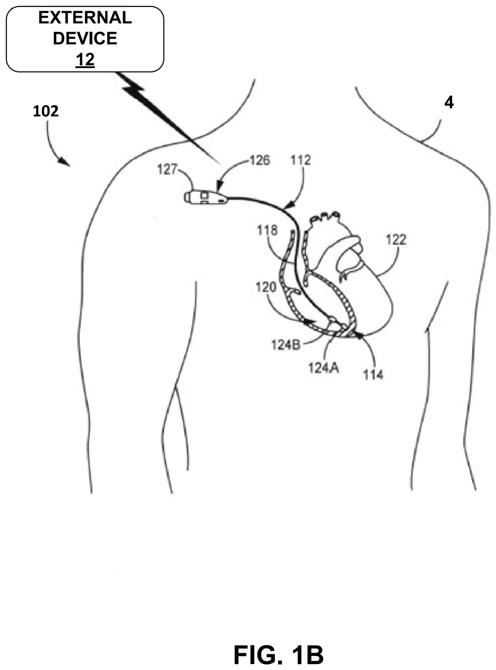

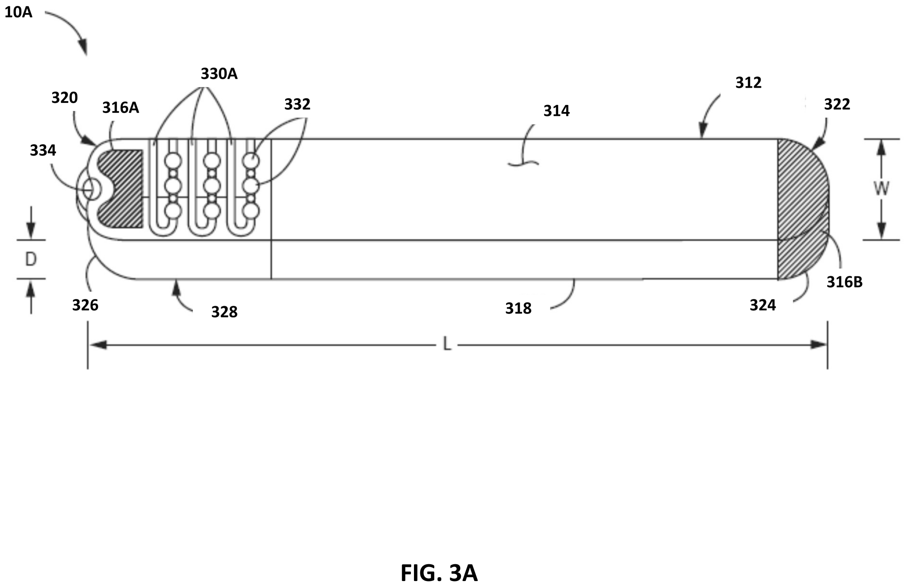

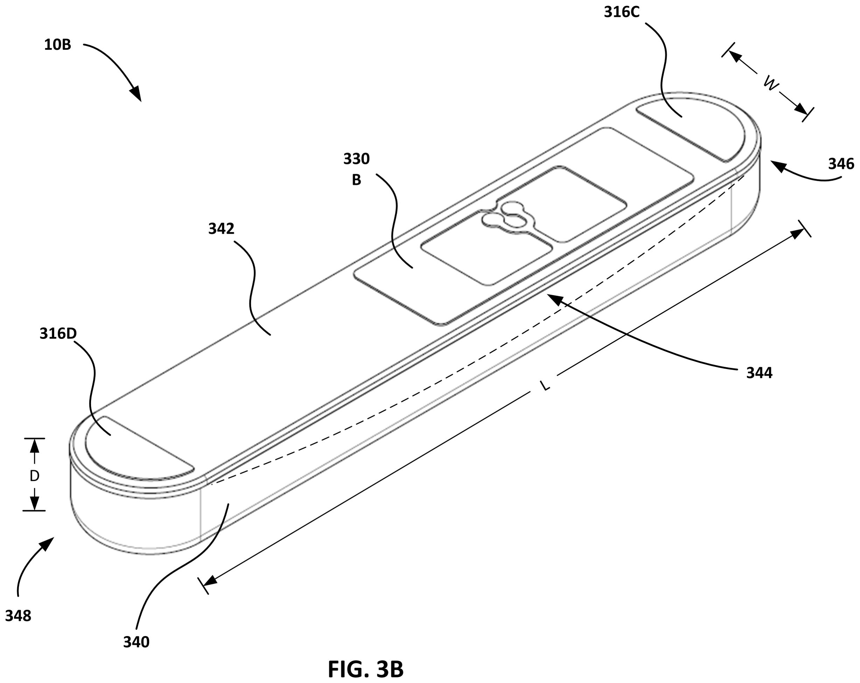

In the example of FIG. 1B, implantable medical device system 102 includes one or more implantable medical leads 112 electrically connected to the IMD 126. The implantable medical lead 112 includes an elongated lead body 118 with a distal portion 120 positioned at a target implantation site 114 within the heart 122 such as, for example, a wall within one or more ventricles or atria. The lead 112 may be a unipolar, a bipolar, or a multipolar lead. IMD 126 may comprise one or more electrodes positioned on housing of IMD 126, such as shown in IMD 10 in FIGS. 3-4.

The implantable medical lead 112 includes one or a plurality of electrodes. In the example of FIG. 1B, the lead 112 includes electrodes 124A and 124B (collectively, "electrodes 124") configured to be positioned on, within, or near cardiac tissue at or near target site 114. In some examples, electrodes 124 provide pacing to heart 122. The electrodes 124 may be electrically connected to conductors (not shown in FIG. 1B) extending through the lead body 118. In some examples, the conductors are electrically connected to therapy delivery circuitry of IMD 126, with the therapy delivery circuitry configured to provide electrical signals through the conductor to electrodes 124. Electrodes 124 may conduct the electrical signals to the target tissue of heart 122, causing the cardiac muscle, e.g., of the ventricles, to depolarize and, in turn, contract at a regular interval. In some examples, IMD 126 may be configured to measure impedance, such as transthoracic impedance, via a vector between at least one of electrodes 124 and at least one electrode positioned on the housing of IMD 126.

Although described primarily in the context of examples in which IMD 10 takes the form of an ICM, the techniques of this disclosure may be implemented in systems including any one or more implantable or external medical devices, including monitors, pacemakers, which may be leadless, e.g., Micra^{™}, cardioverters and/or defibrillators (e.g., subcutaneous, substernal, extravascular, or extracardiac), such as shown in example medical system 102 shown in FIG. 1B. Examples with multiple IMDs or other sensing devices may be able to additional different data useable by system 2 to determine an occurrence of a cough of patient 4 and determine a classification of the cough.

External device 12 may be a computing device with a display viewable by the user and an interface for providing input to external device 12 (i.e., a user input mechanism). External device 12 is configured for wireless communication with IMD 10. External device 12 retrieves sensed physiological data from IMD 10 that was collected and stored by the IMD. In some examples, external device 12 takes the form of a personal computing device of patient 4. For example, external device 12 may take the form of a smartphone of patient 4. In some examples, external device 12 may be any computing device configured for wireless communication with IMD 10, such as a desktop, laptop, or tablet computer. External device 12 may communicate with IMD 10 via near-field communication technologies e.g., inductive coupling, NFC or other communication technologies operable at ranges less than 10-20 cm, and far-field communication technologies, e.g., radiofrequency telemetry according to the Bluetooth^{®} or Bluetooth^{®} Low Energy (BLE) protocols, or other communication technologies operable at ranges greater than near-field communication technologies. When external device 12 is configured for use by the clinician, external device 12 may be used to transmit instructions to IMD 10. The clinician may also configure and store operational parameters for IMD 10 with the aid of external device 12. In some examples, external device 12 assists the clinician in the configuration of IMD 10 by providing a system 2 for identifying potentially beneficial operational parameter values.

External device 12 may be used to retrieve data from IMD 10. The retrieved data may include values of patient parameters measured by IMD 10 based on signals sensed by IMD 10. For example, external device 12 may retrieve physiological signal data, e.g., ECG signal, heart sound signal, respiration signal, BP signal, impedance signal, accelerometer signal, and/or activity/posture signal, on a regular transmission schedule, e.g., daily, due to IMD 10 determining that the patient is coughing or is coughing at a frequency that satisfies a particular threshold, or in response to a request to record the segment from patient 4 or another user. In some examples, one or more sensors of IMD 10 may be enabled simultaneously. In some examples, one or more sensors of IMD 10, such as impedance sensors, may be enabled based on a different signal sensed by IMD 10, such as an ECG signal or heart sound signal. In some examples, IMD 10 may include a sensor configured to sense motion, vibration, and/or orientation with respect to earth gravity, such as an accelerometer configured to sense coughing of patient 4. In some examples, instead or in addition to signal data, external device may retrieve data indicating values of metrics determined by IMD 10 based on the signals, e.g., numbers, frequency, and/or classifications of coughs.

Processing circuitry of system 2, e.g., of IMD 10, external device 12, and/or one or more other computing devices (not illustrated in FIGS. 1A-1B) may be configured to perform the example techniques described herein for determining an occurrence of a cough of patient 4 based on at least one or more physiological signals of patient 4 sensed by IMD 10 and determining a classification of the cough based on the impedance measured by IMD 10. IMD 10 may be configured to measure impedance of patient 4 and sense at least one or more physiological signals of patient 4. In some examples, the measured impedance may indicate at least one of transthoracic impedance or subcutaneous tissue impedance. In some examples, the subcutaneous tissue impedance may be impedance of interstitial fluid. In some examples, the measured impedance may indicate an amount or level of wetness of the lungs of patient 4. For example, the measured transthoracic impedance may indicate an amount of level of wetness of the lungs. In some examples, IMD 10 may be configured to determine transthoracic impedance based on the measured impedance, such as via one or more electrodes on the housing and at least one or more electrodes positioned on lead(s) (not shown in figures), or two or more electrodes on one or more leads, that form a transthoracic measurement vector. In some examples, IMD 10 may be configured to measure transthoracic impedance via at least some of a plurality of electrodes positioned on the housing of the IMD 10. In some examples, IMD 10 may be configured to determine transthoracic impedance based on the measured impedance, via at least some of a plurality of electrodes positioned on the housing of the IMD 10. In some examples, IMD 126 may be configured to determine transthoracic impedance based on a vector between at least one of electrodes 124 and at least one electrode positioned on the housing of IMD 126. In some examples, the physiological signals include at least of an ECG signal and/or an accelerometer signal.

In some examples, processing circuitry of system 2 may be configured to determine an occurrence of a cough of patient 4 based on at least one or more physiological signals of patient 4 as described, for example, in U.S. Patent Publication No. 2021/0106253, incorporated herein by reference in its entirety.

Processing circuitry of system 2 may be configured to determine a classification of a cough based on impedance measured by IMD 10. In some examples, the classifications may include whether the cough is a wet cough or a dry cough. In some examples, the classifications may include whether the cough is a wet cough, an intermediate cough, or a dry cough. In some examples, the classifications may include a likelihood (e.g., score, such as a percentage score) the cough is a wet cough. In some examples, a "wet cough" may indicate that an amount of mucus in the airways satisfies a wet cough threshold. For example, a person may have a wet cough due to mucus in the airways as an attempt to clear mucus from the airways. In some examples, a patient may cough up phlegm or sputum during a wet cough. In some examples, a wet cough may be referred to as a "productive cough" in that the cough is attempting to remove mucus from the airways. In some examples, a presence and/or increase of wet coughs may be an indication of COPD exacerbation and/or COPD worsening.

In some examples, a dry cough may be a cough that is caused by an irritation but may not include significant amounts of phlegm or mucus present in the airways during the cough. In some examples, a dry cough may indicate that an amount of mucus in the airways during a cough does not satisfy a wet cough threshold. In some examples, a dry cough may be due to irritations such as a common cold, smoking, dysphagia or other irritations.

In some examples, processing circuitry of system 2 may be configured to determine a difference between a value of the measured impedance during a first period of time before the cough and a value of the measured impedance during a second period of time after the cough and determine the classification of the cough based on the difference. In some examples, processing circuitry of system 2 may be configured to determine whether the difference satisfies a wet cough threshold and in response to a determination the difference satisfies the wet cough threshold, classify the cough as a wet cough. In some examples, in response to a determination the difference fails to satisfy the wet cough threshold, processing circuitry of system 2 may classify the cough as a dry cough. In some examples, the measured impedance during the first period of time may include one or more of a mean, median, and/or mode of the measured impedance during the first period of time. In some examples, the first period of time is up to 48 hours before the occurrence of the cough. In some examples, the first period of time may be a period of time before a particular cough occurs and the second period of time may be a period of time after a particular cough occurs, such as within 1 minute of the particular cough. In some examples, the difference in impedance may indicate whether a particular wet cough was effective in removing some mucus from the airways. When mucus is removed from the airways of a patient, such as due to an effective cough, the measured impedance, such as the transthoracic impedance, may change due to the change in the amount and/or location of mucus in the airways. In some examples, the difference of measured impedance satisfying the wet cough threshold may indicate the cough removed mucus from the airways which leads to a change in the measured impedance from the first period of time to the second period of time.

In some examples, processing circuitry of system 2 may determine one or more respiration characteristics based on the measured impedance signal. In some examples, the respiration characteristics may include one or more of a respiration rate, tidal volume, cough pattern, mucus extraction (e.g., mucus extracted from lungs), minute ventilation, ratio of volume inspired versus volume expired, and/or difference in volume inspired versus volume expired. In some examples, cough pattern may include one or more of a number of coughs during a particular period of time, differences in periods of each breath with a cough. In some examples, processing circuitry of system 2 may determine one or more changes in respiration characteristics, such as respiration characteristics before a cough and respiration characteristics after a productive cough. In some examples, processing circuitry of system 2 may determine a difference in tidal volume from the first period of time to the second period of time satisfies a wet cough threshold. For example, a large tidal volume change from the first period of time to the second period of time may indicate an effective cough that indicates a wet cough.

In some examples, processing circuitry of system 2 may be configured to determine the measured impedance during the cough and be configured to determine the classification of the cough based on the measured impedance during the cough and determine the classification of the cough based on the measured impedance during the cough. In some examples, processing circuitry of system 2 may be configured to determine whether the measured impedance satisfies a wet cough threshold and in response to a determination that the measured impedance satisfies the wet cough threshold, classify the cough as a wet cough. In some examples, processing circuitry of system 2 may be configured to determine a change or variation in the measured impedance during a cough and determine whether the change or variation in the measured impedance during a cough satisfies a wet cough threshold.

In some examples, processing circuitry of system 2 may be configured to determine the measured impedance during the cough and be configured to determine the respiration pattern of the patient based on the measured impedance during the cough. In some examples, processing circuitry of system 2 may be configured to determine whether the respiration pattern satisfies a wet cough threshold and in response to a determination that the respiration pattern satisfies the wet cough threshold, classify the cough as a wet cough.

In some examples, IMD 10 transmits data to an external device 12, e.g., a smartphone of the patient, configured with an artificial intelligence (AI) application (e.g., the Medtronic My CareLink^{™} Heart) to process the data, track any changes, and notify the clinician and the patient when a visit to clinic is advised or recommended. In some examples, the AI application comprises a machine learning (ML) model. The ML model may be configured to process device data, e.g., impedance signal data, accelerometer data, ECG signal data, heart sound signal data, respiration signal data, BP signal data, and/or activity/posture signal data. In some examples, the device data may include one or more of raw signal data from the device (e.g., respiration waveform) or metrics pre-processed by the device (e.g., respiration rate determined based on, at least, the respiration waveform). The ML model may process the data and compare the results to previous data to determine the occurrence of a cough and/or determining the classification of a cough. In some examples, IMD 10 performs an initial review of the data and determines whether to transmit data to external device 12 for a second review of the data. In some examples, the second review is more complex than the first review. In some examples, both the first and second reviews involve determining the occurrence of a cough and/or determining the classification of a cough.

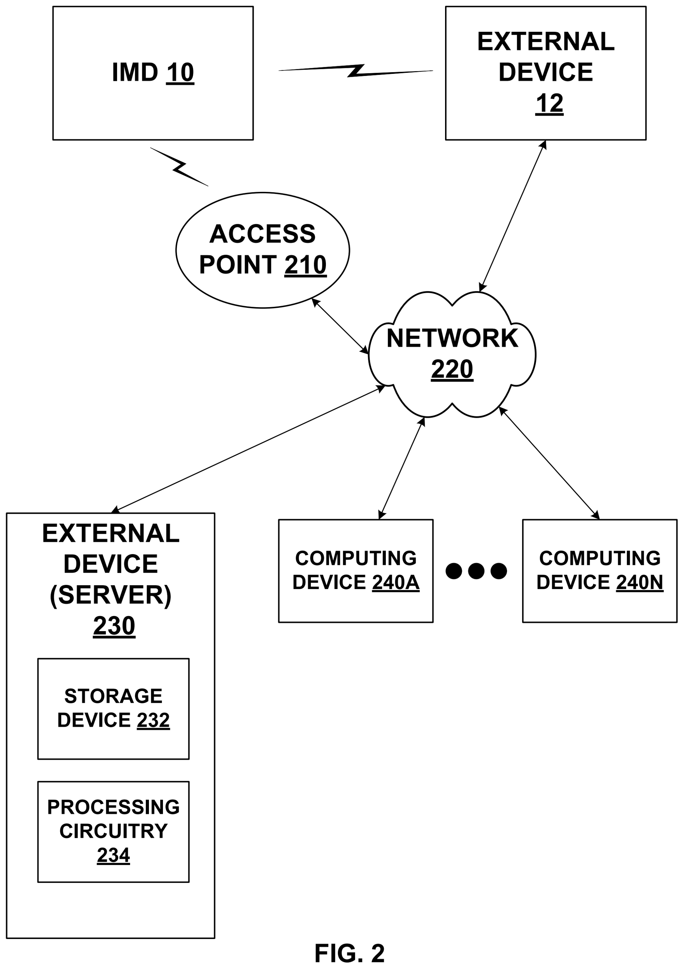

FIG. 2 is a block diagram illustrating an example system that includes an access point 210, a network 220, external computing devices, such as server 230, and one or more other computing devices 240A-240N, which may be coupled to IMD 10, and external device 12 via network 220, in accordance with one or more techniques described herein. IMD 10 may communicate with external device 12 via a first wireless connection and may communicate with an access point 210 via a second wireless connection. In the example of FIG. 2, access point 210, external device 12, server 230, and computing devices 240A-240N are interconnected and may communicate with each other through network 220.

Access point 210 may include a device that connects to network 220 via any of a variety of connections, such as telephone dial-up, digital subscriber line (DSL), or cable modem connections. In other examples, access point 210 may be coupled to network 220 through different forms of connections, including wired or wireless connections. In some examples, access point 90 may be a user device, such as a tablet or smartphone, that may be co-located with the patient. As discussed above, IMD 10 may be configured to transmit physiological data to external device 12. In addition, access point 210 may interrogate IMD 10, such as periodically or in response to a command from the patient or network 220, in order to retrieve patient data from IMD 10. Access point 210 may be communicate the retrieved data to server 230 via network 220.

In some cases, server 230 may be configured to provide a secure storage site for data that has been collected from IMD 10, and/or external device 12. In some cases, server 230 may assemble data for viewing by clinicians via computing devices 240A-240N. One or more aspects of the illustrated system of FIG. 2 may be implemented with general network technology and functionality, which may be similar to that provided by the Medtronic CareLink^{™} Network developed by Medtronic, Inc.

Server 230 may include processing circuitry 234. Processing circuitry 234 may include fixed function circuitry and/or programmable processing circuitry. Processing circuitry 234 may include any one or more of a microprocessor, a controller, digital signal processor (DSP), an application specific integrated circuit (ASIC), a field-programmable gate array (FPGA), graphics processing unit (GPU), tensor processing unit (TPU), or equivalent discrete or analog logic circuitry. In some examples, processing circuitry 234 may include multiple components, such as any combination of one or more microprocessors, one or more controllers, one or more DSPs, one or more ASICs, or one or more FGPAs, one or more GPUs, one or more TPUs, as well as other discrete of integrated logic circuitry. The functions attributed to processing circuitry 234 may be embodied as software, firmware, hardware, or any combination thereof. In some examples, processing circuitry 234 may perform one or more techniques described herein to determine an occurrence of a cough of the patient based on at least one of the physiological signals, such as accelerometer data and/or ECG signals, received from IMD 10 and determine a classification of the cough based on the measured impedance received from IMD 10, as examples.

Server 230 may include storage device 232. Storage device 232 includes computer-readable instructions that, when executed by processing circuitry 234, cause IMD 10 and processing circuitry 234 to perform various functions attributed to IMD 10 and processing circuitry 234 herein. Storage device 232 may include any volatile, non-volatile, magnetic, optical, or electrical media, such as random access memory (RAM), read only memory (ROM), non-volatile RAM (NVRAM), electronically erasable programmable ROM (EEPROM), flash memory, or any other digital media.

In some examples, one or more of computing devices 240A-240N (collectively, "computing devices 240"), e.g., computing device 240A, may be a tablet or other smart device located with a clinician, by which the clinician may program, receive alerts from, and/or interrogate IMD 10. For example, the clinician may access data corresponding to any one or combination of a heart sounds signal, an ECG signal, an activity signal, a respiration signal, a BP signal, an impedance signal, classification of cough, and any other types of signals collected by IMD 10, or metric values or disease state updates determined by IMD 10 based on such signals, through device 240A, such as when patient 4 is in between clinician visits, to check on a status of a medical condition, e.g., of COPD. In some examples, the clinician may enter instructions for medical intervention for patient 4 into an application in computing device 240A, such as based on a status of a patient condition determined by IMD 10, external device 12, or the combination thereof, or based on other patient data known to the clinician. Computing device 240A may then transmit the instructions for medical intervention to another of computing devices 240, e.g., computing device 240B, located with patient 4 or a caregiver of patient 4. For example, such instructions for medical intervention may include an instruction to change a drug dosage, timing, or selection, to schedule a visit with the clinician, or to seek medical attention. In this manner, patient 4 may be empowered to take action, as needed, to address his or her medical status, which may help improve clinical outcomes for patient 4.

FIG. 3A is a perspective drawing illustrating an IMD 10A, which may be an example configuration of IMD 10 of FIG. 1 as an ICM. In the example shown in FIG. 3A, IMD 10A may be embodied as a monitoring device having housing 312, proximal electrode 316A and distal electrode 316B. Housing 312 may further comprise first major surface 314, second major surface 318, proximal end 320, and distal end 322. Housing 312 encloses electronic circuitry located inside the IMD 10A and protects the circuitry contained therein from body fluids. Housing 312 may be hermetically sealed and configured for subcutaneous implantation. Electrical feedthroughs provide electrical connection of electrodes 316A and 316B.

In the example shown in FIG. 3A, IMD 10A is defined by a length *L*, a width *W* and thickness or depth *D* and is in the form of an elongated rectangular prism wherein the length *L* is much larger than the width *W,* which in turn is larger than the depth *D.* In one example, the geometry of the IMD 10A - in particular a width *W* greater than the depth *D* - is selected to allow IMD 10A to be inserted under the skin of the patient using a minimally invasive procedure and to remain in the desired orientation during insertion. For example, the device shown in FIG. 3A includes radial asymmetries (notably, the rectangular shape) along the longitudinal axis that maintains the device in the proper orientation following insertion. For example, the spacing between proximal electrode 316A and distal electrode 316B may range from 5 millimeters (mm) to 55 mm, 30 mm to 55 mm, 35 mm to 55 mm, and from 40 mm to 55 mm and may be any range or individual spacing from 5 mm to 60 mm. In addition, IMD 10A may have a length L that ranges from 30 mm to about 70 mm. In other examples, the length *L* may range from 5 mm to 60 mm, 40 mm to 60 mm, 45 mm to 60 mm and may be any length or range of lengths between about 30 mm and about 70 mm. In addition, the width *W* of major surface 314 may range from 3 mm to 15, mm, from 3 mm to 10 mm, or from 5 mm to 15 mm, and may be any single or range of widths between 3 mm and 15 mm. The thickness of depth *D* of IMD 10A may range from 2 mm to 15 mm, from 2 mm to 9 mm, from 2 mm to 5 mm, from 5 mm to 15 mm, and may be any single or range of depths between 2 mm and 15 mm. In addition, IMD 10A according to an example of the present disclosure is has a geometry and size designed for ease of implant and patient comfort. Examples of IMD 10A described in this disclosure may have a volume of three cubic centimeters (cm) or less, 1.5 cubic cm or less or any volume between three and 1.5 cubic centimeters.

In the example shown in FIG. 3A, once inserted within the patient, the first major surface 314 faces outward, toward the skin of the patient while the second major surface 318 is located opposite the first major surface 314. In addition, in the example shown in FIG. 3A, proximal end 320 and distal end 322 are rounded to reduce discomfort and irritation to surrounding tissue once inserted under the skin of the patient. IMD 10A, including instrument and method for inserting IMD 10A is described, for example, in U.S. Patent Publication No. 2014/0276928, incorporated herein by reference in its entirety.

Proximal electrode 316A is at or proximate to proximal end 320, and distal electrode 316B is at or proximate to distal end 322. Proximal electrode 316A and distal electrode 316B are used to sense ECG signals thoracically outside the ribcage, which may be sub-muscularly or subcutaneously. ECG signals may be stored in a memory of IMD 10A, and data may be transmitted via integrated antenna 330A to another device, which may be another implantable device or an external device, such as external device 12 shown in FIG. 1. In some example, electrodes 316A and 316B may additionally or alternatively be used for sensing any bio-potential signal of interest, which may be, for example, an electroencephalogram (EEG), electromyogram (EMG), or a nerve signal, or for measuring impedance, from any implanted location.

In the example shown in FIG. 3A, proximal electrode 316A is at or in close proximity to the proximal end 320 and distal electrode 316B is at or in close proximity to distal end 322. In this example, distal electrode 316B is not limited to a flattened, outward facing surface, but may extend from first major surface 314 around rounded edges 324 and/or end surface 326 and onto the second major surface 318 so that the electrode 316B has a three-dimensional curved configuration. In some examples, electrode 316B is an uninsulated portion of a metallic, e.g., titanium, part of housing 312.

In the example shown in FIG. 3A, proximal electrode 316A is located on first major surface 314 and is substantially flat, and outward facing. However, in other examples, proximal electrode 316A may utilize the three-dimensional curved configuration of distal electrode 316B, providing a three dimensional proximal electrode (not shown in this example). Similarly, in other examples, distal electrode 316B may utilize a substantially flat, outward facing electrode located on first major surface 314 similar to that shown with respect to proximal electrode 316A.

The various electrode configurations allow for configurations in which proximal electrode 316A and distal electrode 316B are located on both first major surface 314 and second major surface 318. In other configurations, such as that shown in FIG. 3A, only one of proximal electrode 316A and distal electrode 316B is located on both major surfaces 314 and 318, and in still other configurations both proximal electrode 316A and distal electrode 316B are located on one of the first major surface 314 or the second major surface 318 (e.g., proximal electrode 316A located on first major surface 314 while distal electrode 316B is located on second major surface 318). In another example, IMD 10A may include electrodes on both major surface 314 and 318 at or near the proximal and distal ends of the device, such that a total of four electrodes are included on IMD 10A. Electrodes 316A and 316B may be formed of a plurality of different types of biocompatible conductive material, e.g., stainless steel, titanium, platinum, iridium, or alloys thereof, and may utilize one or more coatings such as titanium nitride or fractal titanium nitride.

In the example shown in FIG. 3A, proximal end 320 includes a header assembly 328 that includes one or more of proximal electrode 316A, integrated antenna 330A, anti-migration projections 332, and/or suture hole 334. Integrated antenna 330A is located on the same major surface (i.e., first major surface 314) as proximal electrode 316A and is also included as part of header assembly 328. Integrated antenna 330A allows IMD 10A to transmit and/or receive data. In other examples, integrated antenna 330A may be formed on the opposite major surface as proximal electrode 316A or may be incorporated within the housing 312 of IMD 10A. In the example shown in FIG. 3A, anti-migration projections 332 are located adjacent to integrated antenna 330A and protrude away from first major surface 314 to prevent longitudinal movement of the device. In the example shown in FIG. 3A, anti-migration projections 332 include a plurality (e.g., nine) small bumps or protrusions extending away from first major surface 314. As discussed above, in other examples, anti-migration projections 332 may be located on the opposite major surface as proximal electrode 316A and/or integrated antenna 330A. In addition, in the example shown in FIG. 3A, header assembly 328 includes suture hole 334, which provides another means of securing IMD 10A to the patient to prevent movement following insertion. In the example shown, suture hole 334 is located adjacent to proximal electrode 316A. In one example, header assembly 328 is a molded header assembly made from a polymeric or plastic material, which may be integrated or separable from the main portion of IMD 10A.

FIG. 3B is a perspective drawing illustrating another IMD 10B, which may be another example configuration of IMD 10 from FIG. 1 as an ICM. IMD 10B of FIG. 3B may be configured substantially similarly to IMD 10A of FIG. 3A, with differences between them discussed herein.

IMD 10B may include a leadless, subcutaneously-implantable monitoring device, e.g., an ICM. IMD 10B includes housing having a base 340 and an insulative cover 342. Proximal electrode 316C and distal electrode 316D may be formed or placed on an outer surface of cover 342. Various circuitries and components of IMD 10B may be formed or placed on an inner surface of cover 342, or within base 340. In some examples, a battery or other power source of IMD 10B may be included within base 340. In the illustrated example, antenna 330B is formed or placed on the outer surface of cover 342 but may be formed or placed on the inner surface in some examples. In some examples, insulative cover 342 may be positioned over an open base 340 such that base 340 and cover 342 enclose the circuitries and other components and protect them from fluids such as body fluids. The housing including base 340 and insulative cover 342 may be hermetically sealed and configured for subcutaneous implantation.

Circuitries and components may be formed on the inner side of insulative cover 342, such as by using flip-chip technology. Insulative cover 342 may be flipped onto a base 340. When flipped and placed onto base 340, the components of IMD 10B formed on the inner side of insulative cover 342 may be positioned in a gap 344 defined by base 340. Electrodes 316C and 316D and antenna 330B may be electrically connected to circuitry formed on the inner side of insulative cover 342 through one or more vias (not shown) formed through insulative cover 342. Insulative cover 342 may be formed of sapphire (i.e., corundum), glass, parylene, and/or any other suitable insulating material. Base 340 may be formed from titanium or any other suitable material (e.g., a biocompatible material). Electrodes 316C and 316D may be formed from any of stainless steel, titanium, platinum, iridium, or alloys thereof. In addition, electrodes 316C and 316D may be coated with a material such as titanium nitride or fractal titanium nitride, although other suitable materials and coatings for such electrodes may be used.

In the example shown in FIG. 3B, the housing of IMD 10B defines a length *L*, a width *W* and thickness or depth *D* and is in the form of an elongated rectangular prism wherein the length *L* is much larger than the width *W,* which in turn is larger than the depth *D*, similar to IMD 10A of FIG. 3A. For example, the spacing between proximal electrode 316C and distal electrode 316D may range from 5 mm to 50 mm, from 30 mm to 50 mm, from 35 mm to 45 mm, and may be any single spacing or range of spacings from 5 mm to 50 mm, such as approximately 40 mm. In addition, IMD 10B may have a length L that ranges from 5 mm to about 70 mm. In other examples, the length *L* may range from 30 mm to 70 mm, 40 mm to 60 mm, 45 mm to 55 mm, and may be any single length or range of lengths from 5 mm to 50 mm, such as approximately 45 mm. In addition, the width *W* may range from 3 mm to 15 mm, 5 mm to 15 mm, 5 mm to 10 mm, and may be any single width or range of widths from 3 mm to 15 mm, such as approximately 8 mm. The thickness or depth *D* of IMD 10B may range from 2 mm to 15 mm, from 5 mm to 15 mm, or from 3 mm to 5 mm, and may be any single depth or range of depths between 2 mm and 15 mm, such as approximately 4 mm. IMD 10B may have a volume of three cubic centimeters (cm) or less, or 1.5 cubic cm or less, such as approximately 1.4 cubic cm.

In the example shown in FIG. 3B, once inserted subcutaneously within the patient, outer surface of cover 342 faces outward, toward the skin of the patient. In addition, as shown in FIG. 3B, proximal end 346 and distal end 348 are rounded to reduce discomfort and irritation to surrounding tissue once inserted under the skin of the patient. In addition, edges of IMD 10B may be rounded.

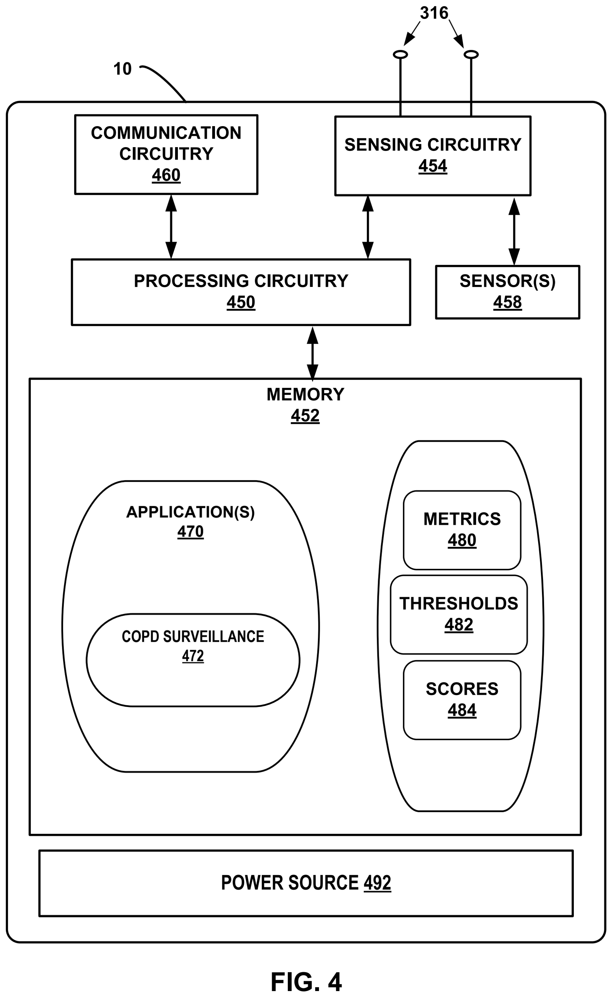

FIG. 4 is a block diagram illustrating an example configuration of an IMD 10 in accordance with one or more techniques described herein. IMD 10 may correspond to either of IMDs 10A and 10B, IMD 126, or another configuration of an IMD. In the illustrated example, IMD 10 includes electrodes 316 (which may correspond to any of electrodes 316A-316D), processing circuitry 450, sensing circuitry 454, sensors 458, communication circuitry 460, power source 492, and memory 452. Although the illustrated example includes two electrodes 316, IMDs including or coupled to more than two electrodes may implement the techniques of this disclosure in some examples. Power source 492 provides operational power for processing circuitry 450, sensing circuitry 454, sensors 458, communication circuitry 460, and memory 452.

Processing circuitry 450 may include fixed function circuitry and/or programmable processing circuitry. Processing circuitry 450 may include any one or more of a microprocessor, a controller, a DSP, an ASIC, a FPGA, a GPU, a TPU, or equivalent discrete or analog logic circuitry. In some examples, processing circuitry 450 may include multiple components, such as any combination of one or more microprocessors, one or more controllers, one or more DSPs, one or more ASICs, or one or more FPGAs, one or more GPUs, one or more TPUs, as well as other discrete or integrated logic circuitry. The functions attributed to processing circuitry 450 herein may be embodied as software, firmware, hardware or any combination thereof.

Sensing circuitry 454 may be coupled to electrodes 316 to sense electrical signals of the heart of patient 4, for example by selecting electrodes 316 and polarity, used to sense an ECG as controlled by processing circuitry 450. Sensing circuitry 454 may sense the ECG from electrodes 316 in order to facilitate monitoring the electrical activity of the heart. In some examples, sensing circuitry 454 may include one or more filters and amplifiers for filtering and amplifying signals received from electrodes 316 and/or sensors 458. Sensing circuitry 454 and processing circuitry 450 may store ECG data in memory 452. In some examples, sensing circuitry 454 may measure impedance, e.g., of tissue in contact with and/or adjacent to IMD 10, and/or of tissue between two or more electrodes 316 forming a sensing vector, via electrodes 316, and store measured impedance values in memory 452. Sensing circuitry 454 may also monitor signals from sensors 458, which may include one or more accelerometers, other vibration or motion sensors, optical sensors, or BP sensors, as examples. Sensing circuitry 454 may capture sensor signals from any one of sensors 458, e.g., to produce other patient data, in order to facilitate monitoring of patient activity and detecting coughs, determining classification of coughs, and/or determining changes in COPD state based on frequency and/or duration of detected coughs and/or the classification of the detected coughs.

Communication circuitry 460 may include any suitable hardware, firmware, software, or any combination thereof for communicating with another device, such as external device 12, another networked computing device, or another IMD or sensor. Under the control of processing circuitry 450, communication circuitry 460 may receive downlink telemetry from, as well as send uplink telemetry to external device 12. In addition, processing circuitry 450 may communicate with a networked computing device via an external device (e.g., external device 12) and a computer network, such as the Medtronic CareLink^{™} Network. Communication circuitry 460 may be configured to transmit and/or receive signals via inductive coupling, electromagnetic coupling, Near Field Communication (NFC), Radio Frequency (RF) communication, Bluetooth, Wi-Fi, or other proprietary or non-proprietary wireless communication schemes.

One or more memory devices 452 may be configured to store information within IMD 10 during operation. Memory device 452, in some examples, is described as a computer-readable storage medium. In some examples, memory device 452 is a temporary memory, meaning that a primary purpose of memory device 452 is not long-term storage. Memory device 452, in some examples, is described as a volatile memory, meaning that memory device 452 does not maintain stored contents when the computer is turned off. Examples of volatile memories include RAM, dynamic RAM (DRAM), static RAM (SRAM), and other forms of volatile memories known in the art. In some examples, memory device 452 is used to store program instructions for execution by processing circuitry 450. Memory device 452, in one example, is used by software or applications 470 running on IMD 10A to temporarily store information during program execution.

Memory device 452, in some examples, also includes one or more non-transitory computer-readable storage media. Memory device 452 may be configured to store larger amounts of information than volatile memory. Memory device 452 may further be configured for long-term storage of information. In some examples, memory device 452 includes non-volatile storage elements. Examples of such non-volatile storage elements include magnetic hard discs, optical discs, floppy discs, flash memories, or forms of electrically programmable memories (EPROM) or EEPROM memories.

Application(s) 470 may include program instructions and/or data that are executable by IMD 10, e.g., by processing circuitry 450. In accordance with the techniques of the disclosure, application(s) 470 includes COPD surveillance 472. Other additional applications not shown may additionally or alternatively be included to provide other functionality described herein and are not depicted for the sake of simplicity.

COPD surveillance 472 is configured to receive metrics 480, thresholds 482, and scores 484, e.g., derived from patient physiological signal data, evaluate the patient metrics, and generate notifications when COPD surveillance 472 determines the COPD state of the patient 4 has changed. Other devices or systems, such as external device 12 or server 230, e.g., of a cloud computing system, may additionally or alternatively implement application(s) 470 and the techniques described herein. In some examples, processing circuitry 450 may control communication circuitry 460 to transmit metric data or signal data used to determine metric data to another device, e.g., external device 12 or a cloud computing system comprising one or more computing devices, for analysis to determine COPD state according to the techniques of this disclosure.

Metrics 480 may include one or more of an impedance signal, an accelerometer signal, an ECG signal, a respiration signal, cough frequency, cough duration, cough classification, or any other signals sensed by IMD 10, or metric values determined based on such signals, as examples. In some examples, metrics 480 may be compared to corresponding thresholds 482, and the comparisons of at least one of metrics 480 to thresholds 482 are used to determine scores 484. In other examples, COPD surveillance 472 determines scores 484 based on a change in metrics 480, e.g., a standard deviation of a frequency of a particular classification of cough or other metric values, over some period of time, or based on a comparison of one or more values of metrics 480 to a baseline meeting a respective criterion, e.g., a threshold criterion. COPD surveillance 472 may compare a threshold 482 to a sum or other combination of scores 484 to determine whether COPD state has changed.

Processing circuitry of 450 may determine one or more of the metrics used to determine COPD state based on a measured impedance of a patient, e.g., patient 4. As an example, one or more of metrics 480 may be determined based on measured impedance measured via electrodes 316. Processing circuitry of 450 may determine one or more of the metrics used to determine COPD state based on an accelerometer signal of a patient, e.g., patient 4. As an example, sensor(s) 458 may be configured to sense an accelerometer signal. Sensor(s) 458 may comprise an accelerometer, or any other device capable of sensing accelerometer signals. Sensing circuitry 454 controls sensor(s) 458, and processing circuitry 450 determines one of more of metrics 480 based on the accelerometer signals. Metrics 480 based on the accelerometer signals may comprise any one of more of cough detection, cough frequency, cough duration, cough magnitude, etc., all of which may be indicative of changes in COPD state.

Additionally or alternatively, one or more metrics 480 may be determined based on an impedance signal. Processing circuitry 450 may determine one or more of the metrics used to determine COPD state based on a measured impedance of a patient, e.g., patient 4. Processing circuitry 450 determines impedance signal information, such as an indication of fluid in the lungs during a detected cough. Processing circuitry 450 may determine one or more metrics 480 based on the impedance information. As an example, one or more of metrics 480 may be determined based on measured impedance measured via electrodes 316. Processing circuitry of 450 may determine a classification of a cough based on the measured impedance. As indicated, a wet cough is a symptom of COPD, but the patient and/ or clinician may not notice a cough is a wet cough or may not notice a duration or frequency of wet coughs over a period of time and therefore may miss an ideal therapy window to intervene in the progression of patient's 4 condition. The techniques of this disclosure may therefore be advantageous to the detection and classification of coughs, which may therefore be advantageous to the detection of a state of COPD monitoring.

As an example, an accelerometer is used to collect activity and heart sound signals, but the techniques of this disclosure are not limited to the use of accelerometers. In other examples, a microphone, pressure transducer, or other devices may be used to collect one or more of the signals described herein. Likewise, as an example, processing circuitry determines respiration signals based on ECG data, but the techniques of this disclosure are not limited to this method of determining respiration signals. In other examples, a pulse oximeter, an accelerometer, impedance measurements via electrodes 316, a pressure sensor, or another sensing device may be used to collect respiration signals.

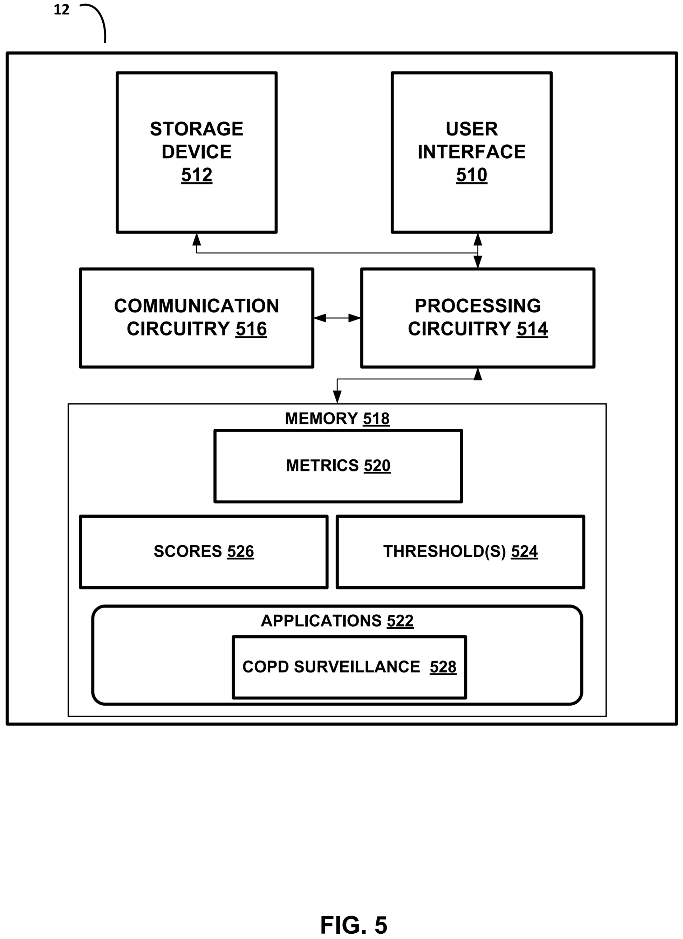

FIG. 5 is a block diagram illustrating an example configuration of components of external device 12. In the example of FIG. 5, external device 12 includes user interface 510, storage device 512, processing circuitry 514, communication circuitry 516, and memory 518.

Processing circuitry 514 may include one or more processors that are configured to implement functionality and/or process instructions for execution within external device 12. For example, processing circuitry 514 may be capable of processing instructions stored in storage device 512. Processing circuitry 514 may include, for example, microprocessors, DSPs, ASICs, FPGAs, GPUs, TPUs, or equivalent discrete or integrated logic circuitry, or a combination of any of the foregoing devices or circuitry. Accordingly, processing circuitry 514 may include any suitable structure, whether in hardware, software, firmware, or any combination thereof, to perform the functions ascribed herein to processing circuitry 514.

Communication circuitry 516 may include any suitable hardware, firmware, software or any combination thereof for communicating with another device, such as IMD 10. Under the control of processing circuitry 514, communication circuitry 516 may receive downlink telemetry from, as well as send uplink telemetry to, IMD 10, or another device. Communication circuitry 516 may be configured to transmit or receive signals via inductive coupling, electromagnetic coupling, NFC, RF communication, Bluetooth, Wi-Fi, or other proprietary or non-proprietary wireless communication schemes. Communication circuitry 516 may also be configured to communicate with devices other than IMD 10 via any of a variety of forms of wired and/or wireless communication and/or network protocols.

Storage device 512 may be configured to store information within external device 12 during operation. Storage device 512 may include a computer-readable storage medium or computer-readable storage device. In some examples, storage device 512 includes one or more of a short-term memory or a long-term memory. Storage device 512 may include, for example, RAM, DRAM, SRAM, magnetic discs, optical discs, flash memories, or forms of EPROM or EEPROM. In some examples, storage device 512 is used to store data indicative of instructions for execution by processing circuitry 514. Storage device 512 may be used by software or applications running on external device 12 to temporarily store information during program execution.

External device 12 may transmit data including computer readable instructions which, when implemented by IMD 10, may control IMD 10 to change one or more operational parameters/device settings and/or export collected data. For example, processing circuitry 514 may transmit an instruction to IMD 10 which requests IMD 10 to export collected data (e.g., metric data 480) to external device 12. In turn, external device 12 may receive the collected data from IMD 10 and store the collected data in storage device 512, e.g., as metric data 520. The data external device 12 receives from IMD 10 may include various patient data such as heart sounds signal, ECG signal, activity signal, respiration signal, BP signal, impedance signal, and/or metric values derived from these signals, and any other patient data. Processing circuitry 514 may implement any of the techniques described herein to analyze the data from IMD 10 for determining an occurrence of a cough, determining a classification of a cough, and/or determining a health condition status, such as COPD status, based, at least in part, on the classification of the detected coughs of patient 4.

Processing circuitry 514 of external device 12, using information contained in memory 518, e.g., metrics 520, applications 522, threshold(s) 524, and scores 526, is operative to analyze data of patient 4 collected by IMD 10 to determine a COPD risk score or determine whether there has been a change in COPD state or risk scores. Applications 522 may include COPD surveillance 528.

Processing circuitry 514 determines one or more values of metrics 520 based on signals that sensors 458 and/or electrodes 316 collect, e.g., impedance signal, measured impedance, accelerometer signal, ECG signal, determination of cough occurrence, respiration pattern, and/or classification of cough(s). COPD surveillance 528 determines scores 526 corresponding to each of metrics 520. In some examples, COPD surveillance 528 determines scores 526 by comparing metrics 520 to threshold(s) 524 corresponding to each of the metrics 520. In other examples, COPD surveillance 528 determines scores 526 based on a change in metrics 520, e.g., a standard deviation of impedance over some time, such as a period of time that includes the occurrence of cough, or based on a comparison of one or more values of metrics 520 to a baseline meeting a respective criterion, e.g., threshold criterion. Processing circuitry 514 then sums or otherwise combines scores 526 and compares the resulting value to a threshold 524 to determine COPD state or risk score.

A user, such as a clinician or patient 4, may interact with external device 12 through user interface 510. User interface 510 includes a display (not shown), such as a liquid crystal display (LCD) or a light emitting diode (LED) display or other type of screen, with which processing circuitry 514 may present information related to IMD 10, e.g., evidence of cough, frequency of cough, duration of cough, magnitude of cough, classification cough, changes in a health condition state, such as COPD, risk scores related to severity of health condition state, such as COPD, and visualizations of various data such as data related to the detected cough(s) and classifications thereof. In addition, user interface 510 may include an input mechanism configured to receive input from the user. The input mechanisms may include, for example, any one or more of buttons, a keypad (e.g., an alphanumeric keypad), a peripheral pointing device, a touch screen, or another input mechanism that allows the user to navigate through user interfaces presented by processing circuitry 514 of external device 12 and provide input. In other examples, user interface 510 also includes audio circuitry for providing audible notifications, instructions, or other sounds to the user, receiving voice commands from the user, or both.

In some examples, system 2 may use alternative or additional devices to IMD 10 to determine an occurrence of a cough and/or determine a classification of the cough. Multiple devices, e.g., two ICMs, an ICM and a leadless cardiac pacemaker, or an ICM and a cardiac pacemaker with a lead may sense respective impedance signals and respective physiological signals.

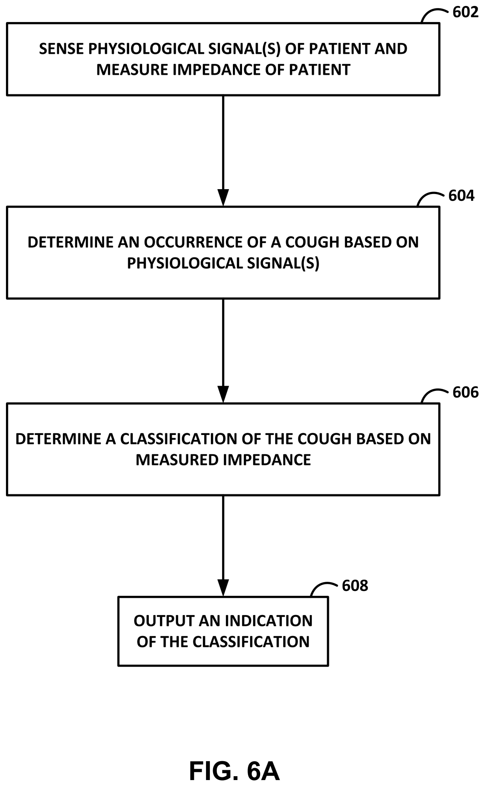

FIGS. 6A-6C are flow diagrams illustrating example operations for determining an occurrence of a cough and determining a classification of the cough. FIGS. 6A-6C are described in the context of an example in which processing circuitry 450 of IMD 10 performs the example operations. In other examples, the operation of FIGS. 6A-6C may be performed in whole or in part by one or more other devices, such as external device 12 and/or server 230.

According to the example of FIG. 6A, IMD 10 senses physiological signals of a patient and measures impedance of a patient, e.g., patient 4 (602). In some examples, IMD 10 may comprise a system capable of collecting impedance signals and collecting accelerometer data and/or ECG data. In some examples, physiological signals may include at least one of an ECG signal or an accelerometer signal. In some examples, the measured impedance indicates at least one of transthoracic impedance or subcutaneous tissue impedance. Processing circuitry 450 may be configured to determine an occurrence of a cough based on the physiological signals (604).

The processing circuitry 450 may be configured to determine a classification of the cough based on the measured impedance (606). In some examples, the classifications may include whether the cough is a wet cough or a dry cough. In some examples, processing circuitry 450 may be configured to output an indication of the classification (608). In some examples, processing circuitry may be configured to output the indication of the classification of the cough to use the classification of the cough as an input to determine a COPD risk score. In some examples, in response to the cough being classified as a wet cough, processing circuitry may be configured use one or more features of the cough that is classified as a wet cough as input to generate a COPD risk score. In some examples, in response to the cough being classified as a dry cough, processing circuitry may be configured to not use the cough or features of the cough that is classified as a dry cough as input to generate a COPD risk score. In some examples, an alert may be output, such as to user(s), indicating the classification of the cough. The alert sent to the user may comprise instructions to a patient, e.g., patient 4, or patient 4's caregiver, to see a clinician for further examination or for immediate medical attention. An alert may additionally or alternatively be sent to a clinician. The alert sent to the clinician may provide more information than was delivered to patient 4. In some cases, a recommended change in medication dosage or type may be provided to the clinician. Additionally, the clinician may receive a risk score related to a COPD state. In some examples, the risk score may be indicative of the level of severity of patient 4's disease state.

The risk score may comprise the COPD score, a comparison between the COPD risk score and a threshold, or some other value based on the COPD risk score. IMD 10, external device 12, or another computing device or cloud computing system may determine the COPD risk score. In some examples, a ML model determines the COPD risk score. In some examples, the clinician may be presented with risk score information along with alerts to changes in disease state via user interface 510 of external device 12. COPD risk score information may additionally comprise information regarding which metric values were included in the calculation. This detailed information may aid the clinician in better interpreting the risk score.

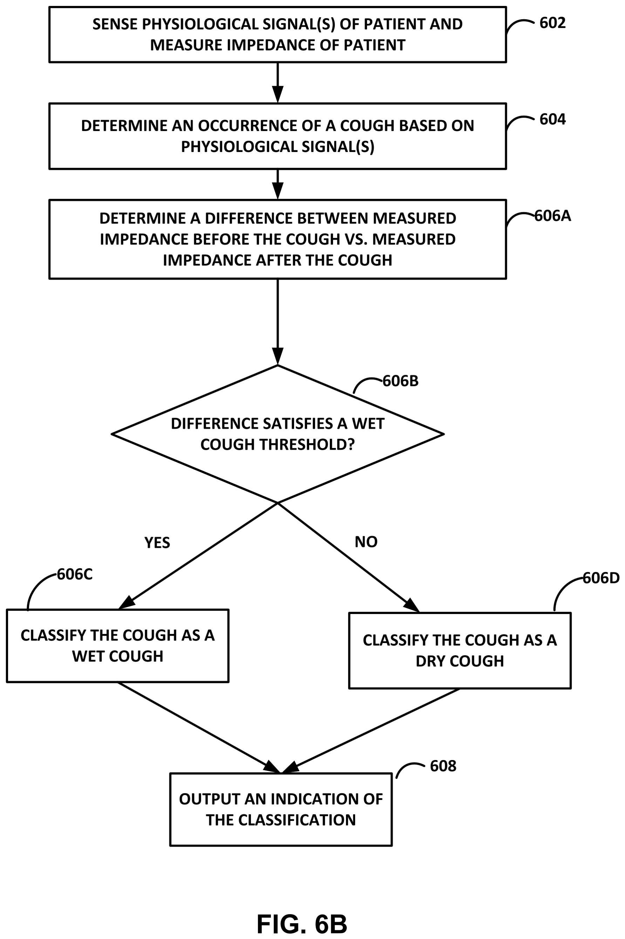

According to the example of FIG. 6B, operations 602, 604, and 608 of FIG. 6B are consistent with operations 602, 604, 608 described above with respect to FIG. 6A. In some examples, processing circuitry 450 may be configured to determine a difference between measure impedance before the cough and the measured impedance after the cough (606A). For example, processing circuitry 450 may be configured to determine a difference between a value of the measured impedance during a first period of time before the cough and a value of the measured impedance during a second period of time after the cough and determine the classification of the cough based on the difference. In some examples, the first period of time may be within 48 hours before the occurrence of the cough. In some examples, the second period of time may be within 5 minutes after the occurrence of the cough. In some examples, the first period of time may be a period of time before a particular cough occurs and the second period of time may be a period of time after a particular cough occurs, such as within 1 minute of the particular cough.

Processing circuitry 450 may be configured to determine whether the difference between a value of the measured impedance during a first period of time before the cough and a value of the measured impedance during a second period of time after the cough satisfies a wet cough threshold (606B). In some examples, the difference in impedance may indicate whether a particular wet cough was effective in removing some mucus from the airways. In some examples, in response to determination the difference satisfies the wet cough threshold ("YES" of 606B), processing circuitry 450 may classify the cough as a wet cough (606C). In some examples, the difference of measured impedance satisfying the wet cough threshold may indicate the cough removed mucus from the airways which leads to a change in the measured impedance from the first period of time to the second period of time. In some examples, in response to determination the difference does not satisfy the wet cough threshold ("NO" of 606B), processing circuitry 450 may classify the cough as a dry cough (606D).

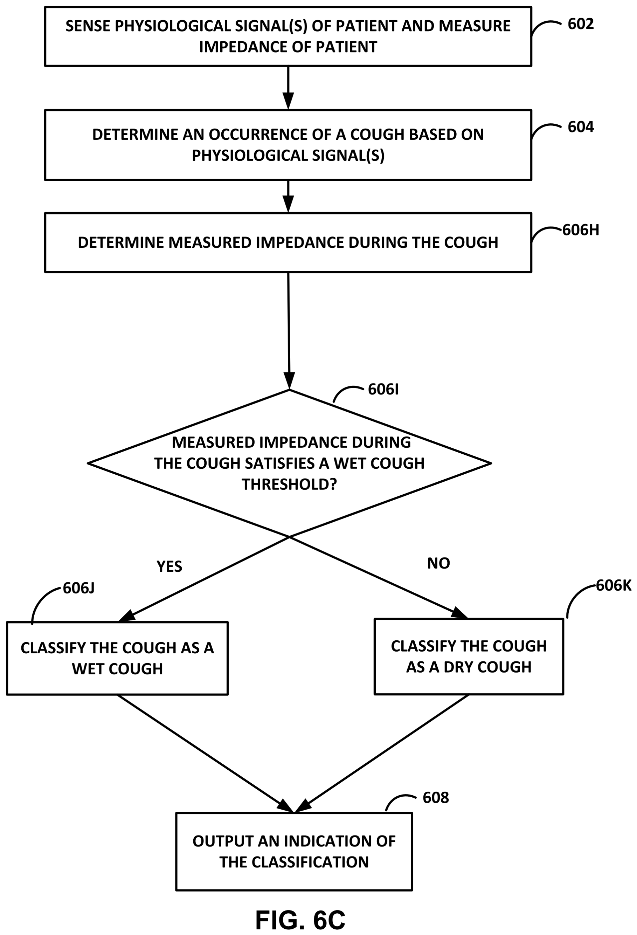

According to the example of FIG. 6C, operations 602, 604, and 608 of FIG. 6B are consistent with operations 602, 604, 608 described above with respect to FIG. 6A. In some examples, processing circuitry 450 may be configured to determine the measured impedance during the occurrence of the cough (606H). Processing circuitry 450 may be configured to determine whether the measured impedance during the cough satisfies a wet cough threshold (606I). In some examples, the measured impedance during the cough may indicate whether a particular wet cough was effective in removing some mucus from the airways. In some examples, processing circuitry 450 may be configured to determine a respiration pattern of the patient based on the measured impedance during the cough. In some examples, processing circuitry 450 may be configured to determine whether the respiration pattern satisfies a wet cough threshold. In some examples, in response to determination the measured impedance during a cough satisfies the wet cough threshold ("YES" of 606I), processing circuitry 450 may classify the cough as a wet cough (606J). In some examples, in response to a determination that the respiration pattern satisfies the wet cough threshold, processing circuitry 450 classify the cough as a wet cough. In some examples, a change or variation of the measured impedance during the cough satisfying the wet cough threshold may indicate the cough removed mucus from the airways which leads to a change or variation in the measured impedance during the cough. In some examples, in response to determination the difference does not satisfy the wet cough threshold ("NO" of 606I), processing circuitry 450 may classify the cough as a dry cough (606K).

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module, unit, or circuit for purposes of clarity, it should be understood that the techniques of this disclosure may be performed by a combination of units, modules, or circuitry associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" or "processing circuitry" as used herein may refer to any of the foregoing structure or any other physical structure suitable for implementation of the described techniques. Also, the techniques could be fully implemented in one or more circuits or logic elements.

The following examples are illustrative of the techniques described herein.

Example 1: A system includes at least one implantable medical device configured to measure impedance and sense at least one or more physiological signals of a patient; and processing circuitry configured to: determine an occurrence of a cough of the patient based on at least one of the physiological signals; determine a classification of the cough based on the measured impedance; and output an indication of the classification of the cough.

Example 2: The system of example 1, wherein to determine the classification of the cough based on the measured impedance, the processing circuitry is configured to: determine a difference between a value of the measured impedance during a first period of time before the cough and a value of the measured impedance during a second period of time after the cough; and determine the classification of the cough based on the difference.

Example 3: The system of example 2, wherein the processing circuitry is further configured to: determine whether the difference satisfies a wet cough threshold; and in response to a determination the difference satisfies the wet cough threshold, classify the cough as a wet cough.

Example 4: The system of example 3, wherein the processing circuitry is further configured to: in response to a determination the difference fails to satisfy the wet cough threshold, classify the cough as a dry cough.

Example 5: The system of any of examples 2-4, wherein the measured impedance during the first period of time is a mean, median, or mode of the measured impedance during the first period of time.

Example 6: The system of any of examples 2-5, wherein the first period of time is up to 48 hours before the occurrence of the cough.

Example 7: The system of example 1, wherein to determine the classification of the cough based on the measured impedance, the processing circuitry is configured to: determine the measured impedance during the cough; and determine the classification of the cough based on the measured impedance during the cough.

Example 8: The system of example 7, wherein the processing circuitry is further configured to: determine whether the measured impedance satisfies a wet cough threshold; and in response to a determination that the measured impedance satisfies the wet cough threshold, classify the cough as a wet cough.

Example 9: The system of example 1, wherein to determine the classification of the cough based on the measured impedance, the processing circuitry is configured to: determine the measured impedance during the cough; and determine a respiration pattern of the patient based on the measured impedance during the cough; determine whether the respiration pattern satisfies a wet cough threshold; and in response to a determination that the respiration pattern satisfies the wet cough threshold, classify the cough as a wet cough.

Example 10: The system of any of examples 1-9, wherein the measured impedance indicates at least one of transthoracic impedance or subcutaneous tissue impedance.

Example 11: The system of any of examples 1-10, wherein the physiological signals include at least of an electrocardiogram (ECG) signal or an accelerometer signal.

Example 12: The system of any of examples 1-3 and 5-11, wherein, in response to the classification of the cough being a wet cough, the processing circuitry is configured to use one or more features of the cough as an input to generate a Chronic Obstructive Pulmonary Disease (COPD) risk score.

Example 13: A method comprising: measuring, by an implantable medical device (IMD), impedance of a patient; sensing, by the IMD, at least one or more physiological signals of the patient; determining, by processing circuitry, an occurrence of a cough of the patient based on at least one of the physiological signals; determining, by the processing circuitry, a classification of the cough based on the measured impedance; and outputting, by the processing circuitry, an indication of the classification of the cough.

Example 14: The method of example 13, wherein determining the classification of the cough based on the measured impedance comprises: determining, by the processing circuitry, a difference between a value of the measured impedance during a first period of time before the cough and a value of the measured impedance during a second period of time after the cough; and determining, by the processing circuitry, the classification of the cough based on the difference.

Example 15: The method of example 14 further comprising: determining, by the processing circuitry, whether the difference satisfies a wet cough threshold; and in response to a determination the difference satisfies the wet cough threshold, classifying, by the processing circuitry, the cough as a wet cough.

Example 16: The method of any of examples 14-15, wherein the measured impedance during the first period of time is a mean, median, or mode of the measured impedance during the first period of time.

Example 17: The method of any of examples 14-16, wherein the first period of time is up to 48 hours before the occurrence of the cough.

Example 18: The method of example 13, wherein determining the classification of the cough based on the measured impedance comprises: determining, by the processing circuitry, the measured impedance during the cough; and determining, by the processing circuitry, the classification of the cough based on the measured impedance during the cough.

Example 19: The method of example 18 further comprising: determining, by the processing circuitry, whether the measured impedance satisfies a wet cough threshold; and in response to a determination the measured impedance satisfies the wet cough threshold, classifying, by the processing circuitry, the cough as a wet cough.

Example 20: The method of example 13, wherein determining the classification of the cough based on the measured impedance comprises: determining, by the processing circuitry, the measured impedance during the cough; and determining, by the processing circuitry, a respiration pattern of the patient based on the measured impedance during the cough; determining, by the processing circuitry, whether the respiration pattern satisfies a wet cough threshold; and in response to a determination that the respiration pattern satisfies the wet cough threshold, classifying the cough as a wet cough.

Example 21: The method of any of examples 13-20, wherein the measured impedance indicates at least one of transthoracic impedance or subcutaneous tissue impedance.

Example 22: The method of any of examples 13-21, wherein the physiological signals include at least of an electrocardiogram (ECG) signal or an accelerometer signal.

Example 23: The method of any of examples 13-22 further comprising: in response to the classification of the cough being a wet cough, using one or more features of the cough as an input to generate a Chronic Obstructive Pulmonary Disease (COPD) risk score.

Various examples have been described. These and other examples are within the scope of the following claims.

## Claims

1. A system comprising:
at least one implantable medical device configured to measure impedance and sense at least one or more physiological signals of a patient; and
processing circuitry configured to:
determine an occurrence of a cough of the patient based on at least one of the physiological signals;
determine a classification of the cough based on the measured impedance; and
output an indication of the classification of the cough.

2. The system of claim 1, wherein to determine the classification of the cough based on the measured impedance, the processing circuitry is configured to:
determine a difference between a value of the measured impedance during a first period of time before the cough and a value of the measured impedance during a second period of time after the cough; and
determine the classification of the cough based on the difference.

3. The system of claim 2, wherein the processing circuitry is further configured to:
determine whether the difference satisfies a wet cough threshold; and
in response to a determination the difference satisfies the wet cough threshold, classify the cough as a wet cough.

4. The system of claim 3, wherein the processing circuitry is further configured to:
in response to a determination the difference fails to satisfy the wet cough threshold, classify the cough as a dry cough.

5. The system of any of claims 2-4, wherein the measured impedance during the first period of time is a mean, median, or mode of the measured impedance during the first period of time.

6. The system of any of claims 2-5, wherein the first period of time is up to 48 hours before the occurrence of the cough.

7. The system of claim 1, wherein to determine the classification of the cough based on the measured impedance, the processing circuitry is configured to:
determine the measured impedance during the cough; and
determine the classification of the cough based on the measured impedance during the cough.

8. The system of claim 7, wherein the processing circuitry is further configured to:
determine whether the measured impedance satisfies a wet cough threshold; and
in response to a determination that the measured impedance satisfies the wet cough threshold, classify the cough as a wet cough.

9. The system of claim 1, wherein to determine the classification of the cough based on the measured impedance, the processing circuitry is configured to:
determine the measured impedance during the cough;
determine a respiration pattern of the patient based on the measured impedance during the cough;
determine whether the respiration pattern satisfies a wet cough threshold; and
in response to a determination that the respiration pattern satisfies the wet cough threshold, classify the cough as a wet cough.

10. The system of any of claims 1-9, wherein the measured impedance indicates at least one of transthoracic impedance or subcutaneous tissue impedance.

11. The system of any of claims 1-10, wherein the physiological signals include at least of an electrocardiogram (ECG) signal or an accelerometer signal.

12. The system of any of claims 1-3 and 5-11, wherein, in response to the classification of the cough being a wet cough, the processing circuitry is configured to use one or more features of the cough as an input to generate a Chronic Obstructive Pulmonary Disease (COPD) risk score.

13. A method comprising:
measuring, by an implantable medical device (IMD), impedance of a patient;
sensing, by the IMD, at least one or more physiological signals of the patient;
determining, by processing circuitry, an occurrence of a cough of the patient based on at least one of the physiological signals;
determining, by the processing circuitry, a classification of the cough based on the measured impedance; and
outputting, by the processing circuitry, an indication of the classification of the cough.

14. The method of claim 13, wherein determining the classification of the cough based on the measured impedance comprises:
determining, by the processing circuitry, a difference between a value of the measured impedance during a first period of time before the cough and a value of the measured impedance during a second period of time after the cough; and
determining, by the processing circuitry, the classification of the cough based on the difference.

15. The method of claim 14 further comprising:
determining, by the processing circuitry, whether the difference satisfies a wet cough threshold; and
in response to a determination the difference satisfies the wet cough threshold, classifying, by the processing circuitry, the cough as a wet cough.
